# EUROPEAN PATENT SPECIFICATION

(11) **EP 0 801 535 B1**
(45) Date of publication and mention of the grant of the patent: **07.05.2003**
(21) Application number: 95944398.7
(22) Date of filing: 28.12.1995
(51) Int. Cl.: A61B 3/16

(54) **APLANATION TONOMETRY APPARATUS**
VORRICHTUNG ZUR APPLANATIONSTONOMETRIE
DISPOSITIF DE TONOMETRIE PAR APLANISSEMENT

(30) Priority: 05.01.1995 IL 11226495
(43) Date of publication of application: 22.10.1997
(73) Proprietor: LIPMAN ELECTRONIC ENGINEERING LTD., 69 719 Tel Aviv (IL)
(72) Inventor: LIPMAN, Aharon, 40297 Michmoret (IL)
(74) Representative: Grünecker, Kinkeldey, Stockmair & Schwanhäusser Anwaltssozietät
(86) International application number: US9516909
(87) International publication number: WO96020635

(56) References cited:
- EP-A- 0 315 160
- WO-A-94/18882
- US-A- 3 338 090
- US-A- 4 523 597
- US-A- 4 987 899
- US-A- 5 070 875
- US-A- 5 190 042
- US-A- 5 203 331
- US-A- 5 355 884

## Description

The present invention relates to tonometry apparatus for measuring the intra-ocular pressure (IOP) of a subject's eye, and particularly to an applanation type tonometer which produces such a measurement by flattening the subject's cornea.

The intra-ocular pressure (IOP), i.e., the pressure within the eyeball, of a person is one of the most important parameters indicating the health status of the person's eye. Various eye diseases cause the IOP to be higher or lower than normal, but it is mainly elevated when the patient is suffering from glaucoma. Glaucoma is an extremely common condition afflicting about 2% of the population over 40 years of age and is one of the major causes of blindness in the world. This disease has no symptoms and is usually diagnosed by tonometry measuring the IOP of the subject. Tonometry is therefore a routine procedure in all eye examinations, especially those of adults.

There are various types of tonometers for measuring the IOP. One type, called an "indentation tonometer", or "impression tonometer", measures the IOP by measuring a deformation produced in the subject's cornea when a constant force is applied. However, the more common type is the "applanation tonometer", which flattens the cornea and measures the force applied. The commonest and most reliable tonometer used at the present time is called the Goldmann applanation tonometer, in which a flat plate is pressed against the subject's cornea, and the area of applanation is viewed by means of a split-lamp and microscope until the diameter of applanation is found to be 3.06 mm. Thus, it was found by Goldmann that at an applanation diameter of 3.06 mm (or an applanation area of 7.35 mm²), the force required to distort the cornea from its convex shape to a flat shape counterbalances the surface tension effect of the tear of the subject, such that when using this applanation diameter, the force in grams multiplied by "10" is directly converted to the IOP in mm of Hg.

In the Goldmann tonometer, the applanation area is measured by optically splitting it into two halves by a biprism, one half being displaced 3.06 mm relative to the other. A fluorescent solution is first applied to the eye to form a ring which is seen as two semi-circles. A dial is then manually rotated to apply a flattening force to the subject's cornea. When the two semi-circular rings touch, the position of the dial, calibrated in mm Hg, indicates the force required to produce an applanation diameter of 3.06 mm.

US Patent Number 4,523,597 describes an apparatus and a method for measuring the intraocular pressure of an eyeball. Therein, the image of a fluorescent ring around the flattened surface of the eyeball is formed to measure the area of the flattened surface, and the intraocular pressure of the eyeball is calculated in accordance with the pressure against the eyeball and the area of the flattened surface corresponding thereto.

US Patent Number 3,338,090 describes a replaceable tip and illuminating means in an applanation tonometer. The tonometer described therein comprises a probe, an optically transparent tip for engaging a surface of an eye and producing an imagine of a meniscus formed about the periphery of contact of said tip, an optical system to transmit light through the probe, illuminating means for receiving the signal from the photosensing means indicating the magnitude of the area of the engaging surface of the tip with the surface of the eye.

A problem with applanation tonometers in general, and the Goldmann applanation tonometer in particular, is the relatively long contact time between the flat plate and the subject's cornea during the measurement. Not only is a long contact time very unpleasant to the subject, but any movement of the subject's eye during the contact time may require restarting the procedure. This long duration of contact generally requires anesthetizing children, and sometimes adults, in order to make the IOP measurements.

Other drawbacks in the use of the existing Goldmann applanation tonometer for making IOP measurements are the dependence or accuracy on the expertise of the person making the measurements, and the large size and bulkiness of the apparatus used for making such measurements.

An object of the present invention is to provide an applanation tonometer having advantages in some or all of the above respects.

According to the present invention, there is provided an applanation tonometer comprising: a light-conducting pressure applicator assembly having an end adapted to be placed in contact with a cornea, of a subject's eye and an opposite end; force applying means for applying a force to the subject's cornea whereby said pressure application assembly displaces it against the subject's cornea thereby flattening a portion thereof; an illuminator for illuminating the subject's cornea; an image transducer at the opposite end of the pressure applicator assembly for receiving therethrough an optical image of at least said portion of the subject's cornea and converting said optical image of at least said portion of the subject's cornea into electrical signals representative thereof; and a data processor for receiving the electrical signals representative of the optical Image and utilizing the optical image as well as information indicative of an amount of force applied to at least said portion of a subject's cornea for producing an output corresponding to the force applied by the pressure applicator assembly to the subject's cornea as determined by the flatness of said portion of said cornea, characterized in that a force applied by force applying means is controllable by an electrical control signal from said data processor in response to the electrical signals received from said image transducer, to produce a predetermined flatness of at least said portion of the subject's cornea, wherein said electrical control signal is operable to be modulated at a frequency substantially higher than a pulse rate of said subject so as to apply a modulated force to the subject's cornea.

According to further features in the preferred embodiments of the invention described below, the pressure applicator, image transducer, and optical system are all incorporated in a common housing which is movable towards the eye to flatten the cornea. Preferably, the illuminator illuminates the subject's cornea with light of predetermined wavelengths, and the optical system includes a light filter passing only the predetermined wavelength to the image transducer.

Two such tonometers are described below for purposes of examples.

In one described tonometer, the force applying means is controlled by an electrical control signal generated by the date processor in response to the electrical signals received from the image transducer to produce a predetermined flatness of the subject's cornea. Such tonometer is thus based on a closed-loop feedback system.

A second tonometer is described wherein the force applying means applies an increasing force to the pressure applicator, and the data processor receiving the electrical signals from the image transducer produces outputs corresponding to the force applied, thereby enabling determining the force applied when a predetermined flatness in the subject's cornea is sensed. Such a tonometer is thus based on an open-loop non-feedback system.

Applanation tonometers constructed with the foregoing features provides a number of important advantages over the conventional Goldmann applanation tonometer. Thus, the time required for making the measurement may be very substantially reduced. In addition, the accuracy of the measurement is not dependent on the expertise of the person making the measurement. In fact. particularly if a light filter of the illuminator bandwidth is used to increase the signal-to-noise ration, it may even be possible to omit the use of fluorescein, which is generally considered mandatory in the conventional Goldmann tonometry. Further, vertical alignment, which is also mandatory in the conventional Goldmann tonometer, is not required using the tonometer of the present invention. Still further, the tonometer of the present invention provides automatic correction for astigmatism.

Still further, where the force applied by the pressure applicator to flatten the cornea is controlled by an electrical control signal generated by the data processor, as in the first described embodiment, the electrical control signal may be modulated at a frequency of many times (at least five times) the pulse rate of the subject, enabling a calculation to be made (e.g., by suing Fourier Transform analysis) of the dependence of the IOP on cardiac pulsation. The phase difference between the applied force and the measured area provides information on the tear viscosity, enabling a correction to be made because of tear viscosity in the calculation of the IOP. Also, by causing the pressure applicator to apply a constant force to the subject's cornea, and measuring the change of IOP with time, information indicating the facility of outflow of the eye fluid is obtainable (Tonography), which information is also useful in assessing the condition of the subject's eye.

Further features and advantages of the invention will be apparent from the description below.

The invention is herein described, by way of example only, with reference to the accompanying drawings, wherein:
**Fig. 1** diagrammatically illustrates the basic elements of one form of applanation tonometer head constructed in accordance with the present invention and a closed-loop feedback system including the applanation tonometer head;
and **Fig. 2** illustrates the applanation tonometer head of Fig. 1 embodied in an open-loop, non-feedback system.

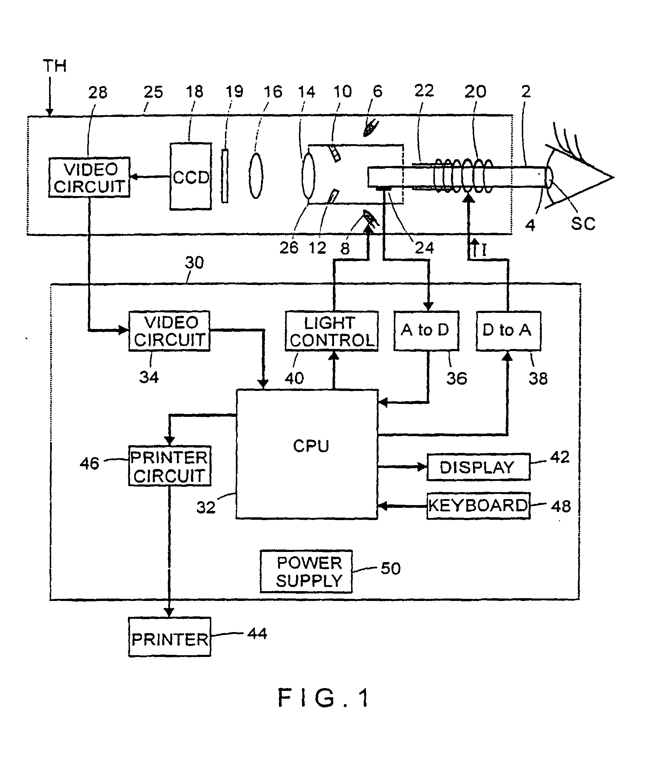

The applanation tonometer head TH illustrated in Fig. 1 comprises a pressure applicator **2** having a flat wall **4** at one end to be pressed into contact with the cornea of the subject's eye, and thereby to flatten the cornea. Pressure applicator **2** may thus be a hollow or solid cylinder of glass or transparent plastic; the flat end wall **4** may be a glass plate. a prism, or biprism made of glass or other transparent material. Flat end wall **4** may be replaceable (for hygienic purposes) or fixed.

The tonometer head TH further includes an illuminator at the end of the pressure applicator **2** opposite to that engaging the subject's cornea, for illuminating the subject's cornea. The illuminator includes a plurality of LEDs (light emitting diodes) **6, 8** at the end of the pressure applicator **2**, and a mirror **10, 12** for each LED to reflect the light therefrom through the pressure applicator **2** to the subject's cornea. The light is reflected back from the subject's cornea through the pressure applicator to a collimating lens **14**. Lens **14** collimates the light to a focussing lens **16**, which focusses the light onto an image transducer **18**. preferably a CCD (charge-coupled device) video camera.

Focussing lens **16** thus focusses the image of the cornea, as engaged by the flat glass plate **4,** onto the image transducer **18.** The image transducer **18** converts the optical image of the subject' cornea into electrical signals.

The LEDs **6, 8** preferably emit short pulses of light (about one microsecond) in a narrow band in the infrared spectrum. A filter **19** is provided between the pressure applicator **2** and the CCD **18** (in this case between lens **16** and the CCD) to pass mainly the predetermined wavelength of the LED light. Such an arrangement minimizes the effects of stray light and thereby increases the signal-to-noise ratio. The wavelength of the light emitted by the LEDs **6**, **8** may be selected to maximize the objects being viewed by the CCD. For example, if it is found that the fluorescent rings are best observed at one specific wavelength, while the contour of the area is best observed at another wavelength, the LEDs would be selected to emit the light including the two wavelengths, and the filter **19** would be selected to maximize the transmission of those wavelengths.

The tonometer head illustrated in Fig. 1 further includes means for applying a force to the pressure applicator **2** for displacing it towards the subject's cornea SC in order to flatten the cornea to a predetermined diameter, namely 3.06 mm when the tonometer is used according to the Goldmann technique. For this purpose, the tonometer head includes an electrical coil **20** enclosing the pressure applicator **2** and magnetically coupled to a metal layer **22** on the outer surface of the pressure applicator to apply a displacing force to the pressure applicator according to the current through the coil. The tonometer head TH further includes a position sensor **24** for measuring the position of the pressure applicator **2**.

The tonometer head TH illustrated in Fig. 1 is incorporated in a common housing **25,** e.g., resembling a pen to be applied against the subject's eye. Collimating lens **14** is mechanically secured to the pressure applicator **2** so that its distance from the end of the pressure applicator remains fixed. This is schematically shown in Fig. 1 by a mechanical connection **26** between lens **14** and the pressure applicator **2**. Since the light exiting from collimating lens **14** is substantially parallel, the filter **19,** focussing lens **16,** and CCD **18** need not, but may be, also mechanically secured to the pressure applicator **2** so as to move with it and with the collimating lens 14.

The electrical signals outputted from the CCD image transducer 18 in the tonometer head TH are processed in a video circuit **28** which may also be within the common housing **25** of the tonometer head.

The electrical output of the video circuit **28** is fed to a data processor, generally designated **30**. Data processor **30** includes CPU (central processor unit) **32** which receives the output of the CCD video circuit **28** via another video circuit **34** within the data processor **30.** The CPU **32** further receives electrical signals from the position sensor **24** via an analog-to-digital converter **36.**

As a result of this inputted information, CPU **32** generates an electrical control signal which is applied via a digital-to-analog converter **38** to the coil **20** to control the current through the coil, and thereby the force applied by the coil to displace the pressure applicator **2**. CPU **32** also outputs an electrical signal to a light control unit **40** which controls the current to the LEDs **6, 8,** and thereby the pulse-duration and intensity of illumination applied to the subject's cornea SC. CPU **32** further outputs information to a display unit **42**, and to a printer **44** via a printer circuit **46**.

Information is inputted into the CPU **32** via a keyboard **48**. The power to all the electrical units illustrated in Fig. 1 is supplied by a power supply **50**.

The tonometer illustrated in Fig. 1 may be used to measure the IOP of the subject's eye according to the Goldmann technique, as follows:

The tonometer head TH is applied to the subject's eye such that one end of the pressure applicator **2** contacts the subject's cornea. Current is then fed via the CPU **32** to coil **20** to apply a force to the pressure applicator **2**, displacing it towards the subject's eye so as to flatten the subject's cornea SC. As this is done, the subject's cornea, illuminated by LEDs **6**, **8** and mirrors **10**, **12**, is imaged via lenses **14**, **16** on the CCD **18**, which outputs electrical signals via video circuits **28**, **34**, to the CPU **32**.

The CPU **32** continuously computes the applanated (flattened) area of the subject's cornea, and controls the current supplied to the coil **20** so as to produce an applanation area of 7.35 mm² (corresponding to an applanation diameter of 3.06 mm). CPU **32** also computes the force required to produce the above applanation area, and outputs this value of force via display **42** and also via printer **44.** The force value is preferably converted by the CPU **32** to mm of Hg.

The system illustrated in Fig. 1 thus provides feedback control of the force applied by the pressure applicator **2** to the subject's cornea to attain and maintain the predetermined flattened area. The IOP can be continuously observed in the display **42,** and/or recorded via the printer **44.** Fluctuations in the IOP caused by the subject's respiration and cardiac pulse can also be observed in a real time manner. These fluctuations thus change the electrical signal outputted by the CPU via the D/A convener **38** to the coil **20** in order to maintain the cornea in the predetermined flattened condition and can therefore be observed in the display **42**.

In addition, applying a constant force, while measuring the change in the IOP with time, provides a measurement of the "facility of outflow" of the eye fluid, which is also useful in determining the condition of the subject's eye. That is, with the applied external force, the IOP rises; this causes the aqueous humour to be driven out of the eye at a rate faster than normal, and consequently the IOP begins to fall. The rate of change depends on the resistance to aqueous outflow.

Further, by modulating the electrical signal applied to winding **20**, a modulated force is applied by the pressure applicator **2** to the subject's cornea. When such a modulated force is applied, the dependence of the IOP on ocular pulsation may be calculated using, for example, Fourier Transform analysis. Moreover, the phase difference between the applied force and the measured area provides information on the tear viscosity. Since calculation of the IOP depends on tear viscosity, this information enables a correction to be made.

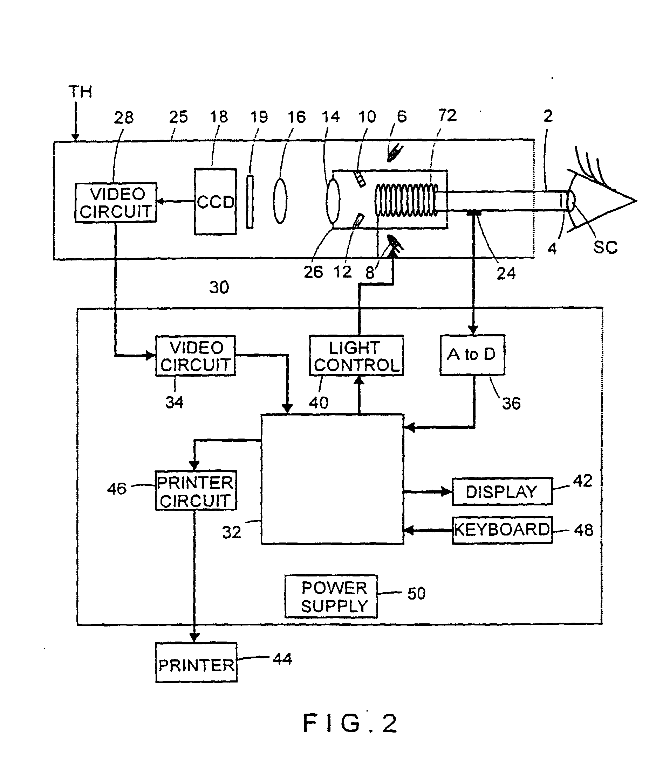

Fig. 2 illustrates a tonometer similar to that described above with respect to Fig. 1, except that the tonometer is included in an open-loop, non-feedback system, rather than in a closed-loop, feedback system as described with respect to Fig. 1. In order to facilitate understanding, the same parts corresponding to those described with respect to Fig. 1 carry the same reference numerals.

A main difference in the tonometer illustrated in Fig. 2, is that a continuously-increasing force is applied to the pressure applicator **2** tending to flatten the subject's cornea SC, and the data processor produces outputs corresponding to the force applied. Thus when the subject's cornea attains the predetermined flatness (an area of 7.35 mm² according to the Goldmann procedure as described above), as viewed by the CCD **18,** the output of the CPU **32** at that time corresponds to the force applied to the pressure applicator at that time.

The pressure applicator 2 may be subjected to a continuously increasing force in any convenient manner, either manually or by machine. For this purpose, the tonometer head TH is provided with a spring **72**. One end of the spring is fixed with respect to the housing **25** of the tonometer head TH, whereas the other end bears against the end of the pressure applicator **2**. Thus, as the tonometer head is moved, either manually or by machine, towards the subject's eye, the pressure applied by the pressure applicator **2** against the subject's cornea will be increased by the compression of spring **72.**

The position sensor **24** generates a signal corresponding to the position of the pressure applicator **2** within the housing **25**. This signal is outputted to the CPU **32** via the analog-to-digital converter **36**. Since the parameters of spring **72** are known, and the displacement of the pressure applicator **2** is known by the position sensor **24,** the CPU can thereby compute the force applied to the pressure applicator when the flattened area, as viewed by the CCD **18**. reaches the predetermined value.

The system illustrated in Fig. 2 is otherwise constructed, and operates, in substantially the same manner as described above with respect to Fig. 1.

While the invention has been described with respect to two preferred embodiments, it will be appreciated that these are set forth merely for purposes of example, and that many variations, modifications and other applications of the invention may be made thin the scope of the claims.

## Claims

1. An Applanation tonometer comprising:
a light-conducting pressure applicator assembly having an end (4) adapted to be placed in contact with the cornea of a subject's eye and an opposite end;
force applying means (72) for applying a force to the subject's cornea (SC) whereby said pressure applicator assembly displaces it against the subject's cornea (SC) and thereby to flatten a portion thereof;
an illuminator (6,8) for illuminating the subject's cornea (SC);
an image transducer (18) at the opposite end of the pressure applicator assembly for receiving therethrough an optical image of at least said portion of the subject's cornea (SC) and converting said optical image of at least said portion of the subject's cornea (SC)into electrical signals representative thereof; and
a data processor (32) for receiving the electrical signals representative of the optical image and utilizing the optical image as well as information indicative of an amount of force applied to at least said portion of the subject's cornea (SC) for producing an output corresponding to the force applied by the pressure applicator assembly to the subject's cornea (SC) as determined by the flatness of said portion of said cornea,
**characterized in that**
a force applied by force applying means (72) is controllable by an electrical control signal from said data processor (32) in response to the electrical signals received from said image transducer (18), to produce a predetermined flatness of at least said portion of the subject's cornea (SC),
wherein said electrical control signal is operable to be modulated at a frequency substantially higher than a pulse rate of said subject so as to apply a modulated force to the subject's cornea (SC).

2. A tonometer according to claim 1, wherein the pressure applicator assembly comprises a collimating optical system (14) located at said opposite end.

3. A tonometer according to claim 1 or claim 2, wherein said illuminator (6, 8) is operable to illuminate at least part of the subject's cornea (SC) with light of a predetermined wavelength, said tonometer also comprising a light filter (19) passing at least mainly said predetermined wavelength to said image transducer (18).

4. A tonometer according to claim 1, wherein said force applying means (72) comprises a metal layer on said pressure applicator assembly and an electrical coil enclosing said pressure applicator assembly and magnetically coupled to said metal layer.

5. A tonometer according to any one of claims 2 to 4, wherein said force applying means (72) is operable to apply an increasing force to at least said portion of said subject's cornea (SC).

6. A tonometer according to claim 5, wherein said force applying means (72) comprises a spring having one end fixed and an opposite end bearing against said opposite end of the pressure applicator assembly.

7. A tonometer according to claim 6, further including a position sensor (24) for sensing the position of the pressure applicator assembly and for outputting a second electrical signal corresponding thereto to said data processor (32), said data processor (32) further being able to utilize said second electrical signal for producing an output corresponding to the force applied to at least said portion of said subject's cornea (SC).

8. A tonometer according to any preceding claim, wherein said image transducer (18) comprises a charge coupled device (CCD).

9. A tonometer according to any preceding claim, wherein said illuminator (6, 8) includes a light emitting diode (LED) and a mirror (10, 12) for reflecting the light therefrom into said opposite end of the pressure applicator assembly.

10. A tonometer according to claim 9, wherein said LED is energized by a plurality of short pulses.

11. A tonometer according to any preceding claim, wherein said collimating optical system (14) comprises a collimating lens fixed to said pressure applicator assembly the tonometer also comprising a focusing lens (16) between said collimating lens and said image transducer (18) for focusing the image of at least said portion of the subject's cornea (SC) onto said image transducer (18).

12. A tonometer according to any preceding claim, wherein said data processor (32) includes an input keyboard (48) and an output printer (44).

## Patentansprüche

1. Applanationstonometer, enthaltend:
eine lichtleitende Druckzuführungsanordnung, die ein Ende (4) hat, das dazu eingerichtet ist, mit der Hornhaut eines Patientenauges in Berührung gebracht zu werden, und ein entgegengesetztes Ende hat;
eine Kraftzuführungseinrichtung (72) zum Aufbringen einer Kraft auf die Patientenhornhaut (SC), wodurch die Druckzuführungsanordnung gegen die Patientenhornhaut (SC) verstellt wird und sie dadurch einen Teil derselben abflacht;
einen Beleuchter (6,8) zum Beleuchten der Patientenhomhaut (SC);
einen Bildwandler (18) am entgegengesetzten Ende der Druckzuführungsanordnung zum Aufnehmen eines optischen Abbildes durch diesen hindurch von wenigstens dem genannten Abschnitt der Patientenhornhaut (SC) und zum Umwandeln dieses optischen Abbildes des genannten Abschnitts der Patientenhomhaut (SC) in dafür repräsentative elektrische Signale; und
einen Datenprozessor (32) zum Aufnehmen der elektrischen Signale, die für das optische Abbild repräsentativ sind, und zum Verwenden des optischen Abbildes und sowie von Information, die die Größe der Kraft angibt, die auf den genannten Abschnitt der Patientenhomhaut (SC) aufgebracht wird, zur Erzeugung einer Ausgabe entsprechend der durch die Druckzuführungsanordnung auf die Patientenhornhaut (SC) aufgebrachten Kraft, wie sie durch die Flachheit des genannten Homhautabschnitts bestimmt ist,
**dadurch gekennzeichnet, dass**
eine von der Kraftzuführungseinrichtung (72) zugeführte Kraft durch ein elektrisches Steuersignal von dem Datenprozessor (32) in Abhängigkeit von den elektrischen Signalen beeinflussbar ist, die von dem Bildwandler (18) empfangen werden, um eine vorbestimmte Flachheit von wenigstens dem genannten Abschnitt der Patientenhornhaut (SC) zu erzeugen,
wobei das elektrische Steuersignal so aufbereitbar ist, dass es mit einer Frequenz moduliert wird, die wesentlich höher als der Puls des Patienten ist, um auf die Patientenhornhaut eine modulierte Kraft aufzubringen.

2. Tonometer nach Anspruch 1, bei dem die Druckzuführungsanordnung ein optisches Kollimatorsystem (14)enthält, das an dem genannten entgegengesetzten Ende angeordnet ist.

3. Tonometer nach Anspruch 1 oder 2, bei dem der Beleuchter (6,8) so betreibbar ist, dass er wenigstens einen Teil der Patientenhornhaut (SC) mit Licht einer vorbestimmten Wellenlänge beleuchtet, wobei das Tonometer auch ein Lichtfilter (19) enthält, das wenigstens hauptsächlich die vorbestimmte Wellenlänge zum Bildwandler (18) durchlässt.

4. Tonometer nach Anspruch 1, bei dem die Kraftzuführungseinrichtung (72) eine Metallschicht auf der Druckzuführungsanordnung und eine die Druckzuführungsanordnung umschließende elektrische Spule, die mit der Metallschicht magnetisch gekoppelt ist, enthält.

5. Tonometer nach einem der Ansprüche 2 bis 4, bei dem die Kraftzuführungseinrichtung (72) so betreibbar ist, dass sie eine zunehmende Kraft auf wenigstens den genannten Abschnitt der Patientenhomhaut (SC) aufbringt.

6. Tonometer nach Anspruch 5, bei dem die Kraftzuführungseinrichtung (72) eine Feder enthält, von der ein Ende festgelegt und ein entgegensetztes Ende sich am entgegengesetzten Ende der Druckzuführungsanordnung abstützt.

7. Tonometer nach Anspruch 6, weiterhin enthaltend einen Positionssensor (24) zur Erfassung der Position der Druckzuführungsanordnung und zum Ausgeben eines zweiten, dementsprechenden Signals an den Datenprozessor (32), wobei dieser weiterhin in der Lage ist, dieses zweite elektrische Signal zum Erzeugen eines Ausgangs zu verwenden, der der Kraft entspricht, die wenigstens auf den genannten Abschnitt der Patientenhornhaut (C) einwirkt.

8. Tonometer nach einem der vorhergehenden Ansprüche, bei dem der Bildwandler (18) eine ladungsgekoppelte Vorrichtung (CCD) enthält.

9. Tonometer nach einem der vorhergehenden Ansprüche, bei dem der Beleuchter (6,8) eine Leuchtdiode (LED) und einen Spiegel (10,12) zum Reflektieren des Lichts davon in das entgegengesetzte Ende der Druckzuführungsanordnung enthält.

10. Tonometer nach Anspruch 9, bei dem die LED durch eine Vielzahl kurzer Impulse erregt wird.

11. Tonometer nach einem der vorhergehenden Ansprüche, bei dem das optische Kollimatorsystem (14) eine Kollimatorlinse enthält, die an der Druckzuführungsanordnung befestigt ist, wobei das Tonometer weiterhin eine Fokussierlinse (16) zwischen der Kollimatorlinse und dem Bildwandler (18) zum Fokussieren des Abbildes von wenigstens dem Abschnitt der Patientenhornhaut (SC) auf den Bildwandler (18) enthält.

12. Tonometer nach einem der vorhergehenden Ansprüche, bei dem der Datenprozessor (32) eine Eingabetastatur (48) und einen Ausgabedrucker (44) aufweist.

## Revendications

1. Tonomètre par aplanation comprenant :
un dispositif d'application de pression conducteur de lumière ayant une extrémité (4) adaptée pour être placée au contact de la cornée de l'oeil d'un sujet et une extrémité opposée ;
des moyens d'application de force (72) pour appliquer une force sur la cornée du sujet (SC), par lequel ledit dispositif applicateur de pression le déplace contre la cornée du sujet (SC) pour aplanir une partie de celle-ci ;
un illuminateur (6, 8) pour illuminer la cornée du sujet (SC) ;
un transducteur d'image (18) situé à l'extrémité opposée du dispositif d'application de pression pour recevoir, par son intermédiaire, une image optique d'au moins ladite partie de la cornée du sujet (SC) et convertir ladite image optique d'au moins ladite partie de la cornée du sujet (SC) en des signaux électriques représentatifs de celle-ci ; et
un processeur de données (32) pour recevoir les signaux électriques représentatifs de l'image optique et utiliser l'image optique ainsi que les informations indicatives d'une grandeur de force appliquée à au moins ladite partie de la cornée du sujet (SC) pour produire une sortie correspondant à la force appliquée par le dispositif d'application de pression à la cornée du sujet (SC), telle que déterminée par la planéité de ladite partie de ladite cornée,
**caractérisé en ce que**
une force appliquée par les moyens d'application de force (72) est contrôlable par un signal de commande électrique provenant dudit processeur de données (32) en réponse aux signaux électriques reçus dudit transducteur d'image (18), pour produire une planéité prédéterminée d'au moins ladite partie de la cornée du sujet (SC),
dans lequel ledit signal de commande électrique est exploitable pour être modulé à une fréquence sensiblement plus élevée qu'un taux d'impulsions dudit sujet de façon à appliquer une force modulée à la cornée du sujet (SC).

2. Tonomètre selon la revendication 1, dans lequel le dispositif d'application de pression comprend un système optique de collimation (14) situé au niveau de ladite extrémité opposée.

3. Tonomètre selon la revendication 1 ou la revendication 2, dans lequel ledit illuminateur (6, 8) est exploitable pour illuminer au moins une partie de la cornée du sujet (SC) avec une lumière d'une longueur d'onde prédéterminée, ledit tonomètre comprenant également un filtre de lumière (19) laissant passer au moins principalement ladite longueur d'onde prédéterminée vers ledit transducteur d'image (18).

4. Tonomètre selon la revendication 1, dans lequel lesdits moyens d'application de force (72) comprennent une couche de métal sur ledit dispositif d'application de pression et une bobine électrique entourant ledit dispositif d'application de pression et couplée magnétiquement à ladite couche de métal.

5. Tonomètre selon l'une des revendications 2 à 4, dans lequel lesdits moyens d'application de force (72) sont exploitables pour appliquer une force croissante sur au moins ladite partie de la cornée dudit sujet (SC).

6. Tonomètre selon la revendication 5, dans lequel lesdits moyens d'application de force (72) comprennent un ressort ayant une extrémité fixée et une extrémité opposée en appui contre ladite extrémité opposée du dispositif d'application de pression.

7. Tonomètre selon la revendication 6, comprenant en outre un capteur de position (24) pour capter la position du dispositif d'application de pression et pour émettre un second signal électrique correspondant à celle-ci audit processeur de données (32), ledit processeur de données (32) étant en outre capable d'utiliser ledit second signal électrique pour produire une sortie correspondant, à la force appliquée à au moins ladite partie de la cornée dudit sujet (SC).

8. Tonomètre selon l'une des revendications précédentes, dans lequel ledit transducteur d'image (18) comprend un dispositif à couplage de charges (CCD).

9. Tonomètre selon l'une des revendications précédentes, dans lequel ledit illuminateur (6, 8) comprend une diode électroluminescente (DEL) et un miroir (10, 12) pour réfléchir la lumière issue de celle-ci dans ladite extrémité opposée du dispositif d'application de pression.

10. Tonomètre selon la revendication 9, dans lequel ladite DEL est alimentée par une pluralité de courtes impulsions.

11. Tonomètre selon l'une des revendications précédentes, dans lequel ledit système optique de collimation (14) comprend une lentille de collimation fixée audit dispositif d'application de pression, le tonomètre comprenant également une lentille de mise au point (16) entre ladite lentille de collimation et ledit transducteur d'image (18) pour focaliser l'image d'au moins une partie de la cornée du sujet (SC) sur ledit transducteur d'image (18).

12. Tonomètre selon l'une des revendications précédentes, dans lequel ledit processeur de données (32) comprend un clavier d'entrée (48) et une imprimante de sortie (44).
